# EUROPEAN PATENT SPECIFICATION

(11) **EP 4 473 083 B1**
(45) Date of publication and mention of the grant of the patent: **31.12.2025**
(21) Application number: 22814011.7
(22) Date of filing: 07.11.2022
(51) Int. Cl.: C12M 1/107, C12M 1/12, C12M 1/00, C12P 5/02

(54) **METHOD AND APPARATUS FOR BIOLOGICAL PRODUCTION OF ELECTRO-METHANE**
VERFAHREN UND VORRICHTUNG ZUR BIOLOGISCHEN HERSTELLUNG VON ELEKTROTREIBSTOFF
PROCÉDÉ ET APPAREIL POUR LA FABRICATION BIOLOGIQUE D'ÉLECTRO-CARBURANT

(30) Priority: 04.02.2022 EP 22155126
(43) Date of publication of application: 11.12.2024
(73) Proprietor: Biogasclean A/S, 5220 Odense SØ (DK)
(72) Inventor: THYGESEN, Peter, 5220 Odense SØ (DK); THOMSEN, Jan, 5220 Odense SØ (DK)
(74) Representative: Plougmann Vingtoft a/s
(86) International application number: PCT/EP2022/081022
(87) International publication number: WO 2023/147905

(56) References cited:
- EP-B1- 2 675 904
- EP-B1- 3 013 937
- DE-A1- 3 130 991
- JP-A- 2004 041 929
- US-A1- 2010 273 242
- US-B2- 11 078 097

## Description

### Technical field of the invention

The present invention relates to a method and apparatus for efficiently converting carbon monooxide and/or carbon dioxide and hydrogen in a gas to methane. The gas comprising carbon monooxide and/or carbon dioxide is preferably a biogas, fluegas or syngas. The gas comprising hydrogen is preferably a gas obtained from electrolysis of water. In particular, the present invention relates to a method and apparatus for converting carbon monooxide and/or carbon dioxide and hydrogen in a gas to methane comprising efficiently cleaning of the packaging material, without removing the packaging material from the reactor, in order to optimise and maintain the efficiency of the conversion. In addition, the present invention relates to converting hydrogen generated from electrolysis of water or similar technologies powered by renewable energy sources to a renewable and storable energy such as methane. Furthermore, the present invention relates in an embodiment to increasing the amount of green energy obtained from anaerobic digestion of an organic material by reducing the content of carbon dioxide and increase the content of methane in a biogas. Hereby, the amount of carbon dioxide emitted to the environment is also reduced.

### Background of the invention

The rapid growth of the world's population causes drastic increase in energy demand. Currently, the main energy supply is from carbon-containing fossil fuel sources like coal, oil and natural gas. However, the energy sources produced from non-renewable type of energy are said to be depleted soon. Apart from this, the fossil fuels need to be combusted to obtain the energy needed. The combustion of fossil fuels contributes to the emission of the largest greenhouse gasses like carbon dioxide into the atmosphere and global warming.

Throughout the world there is an increasing focus on protecting the environment, and many countries have therefore moved toward the generation of a cleaner and greener energy as an alternative energy source, such as exploiting of renewable energy sources. An example of an alternative renewable energy source that can be used is biogas since biogas comprises methane that can be used for energy generation. Biogas is a by-product obtained from digesting organic material in an anaerobic digester. Biogas is a renewable energy source like wind power and solar power. However, biogas is not only produced to provide renewable energy, the production of biogas is an integral part of sustainable treatment of organic waste from the production of the many products that are the prerequisite for our modern life. Furthermore, biogas has, as compared to wind and solar energy, the advantage that it can be produced all year round regardless of weather conditions. On the contrary, wind and solar energy is dependent of either the wind blowing or the sun shining. Further, biogas has the advantage of being able to be stored.

The organic material treated in the anaerobic digesters is called substrate and can derive from a number of different sources including manure from animals, such as pigs, cattle and chicken, food processing plants, breweries, palm oil mils, starch factories, ethanol plants, paper mills, municipal sewage treatment plants, sorted household waste, etc. The treatment in the anaerobic digester removes many of the harmful components present in the waste so that after treatment, the digested water and biomass can be discharged and/or beneficial used for fertilization and irrigation.

From the anaerobic digestion process, a gaseous by-product known as biogas is created. A biogas typically comprises 50-70% methane (CH₄), 30-50% carbon dioxide (CO₂) and about 0.1-5.0% hydrogen sulphide (H₂S).

The biogas can be a significant resource as a renewable substitute for fossil fuels. However, before being used as an energy source, the biogas needs to be cleaned from unwanted compounds. For example, before the biogas can be used in boilers and engines, hydrogen sulphide should be removed to avoid corrosion of equipment and because hydrogen sulphide is toxic to humans. Removal of hydrogen sulphide from a biogas is for example disclosed in European patent application EP 3 487 606 A1. If the biogas is used directly for power generation in combustion engines or boilers, it is not necessary to remove the carbon dioxide. However, if the biogas is used as a replacement of natural gas, it is necessary to remove or at least reduce the carbon dioxide content and "upgrade" the biogas to bio-methane to meet the specifications for natural gas. This is also known as Renewable Natural Gas (RNG).

Some technologies are known that relate to removing or reducing the carbon dioxide content in a gas. For example absorption by chemical solvents, physical absorption by water scrubbing, cryogenic separation, membrane separation and CO₂ fixation by biological or chemical methods. For example, EP 2 032 709 B1 discloses a method of converting CO₂ and H₂ into methane and water by using methanogenic microorganisms in a bioreactor. The methanogenic microorganisms are grown in the bioreactor/fermentor vessel in a suitable liquid culture medium and the gasses comprising CO₂ and H₂ is bubbled through the liquid. However, the conversion of carbon dioxide and hydrogen into methane and water by bubbling a gas through a liquid has some disadvantages, such as; 1) the gas comprising carbon dioxide and the gas comprising hydrogen distribute poorly throughout the liquid, 2) hydrogen gas is poorly soluble in water, and 3) the growth of the microorganisms free flowing in a liquid is not optimal.

EP 3 013 937 B1 discloses a method and apparatus of biomethanation by converting H₂ and CO₂ to methane in a reactor by using methanogenic microorganisms. The method and apparatus comprises a reactor comprising an aqueous medium and the microorganisms are located in the aqueous medium. EP 3 013 937 describes a reaction boosting device and that said reaction boosting device for example can be supplying energy by means of mechanically dynamic means such as nozzles, stirrers and the like to provide mixing or stirring of the aquous medium with the substrate gas.

JP 2004 041929 A discloses a methane fermentation apparatus comprising a fermentation tank comprising microorganisms supported on a carrier.

US 2010/273242 A discloses a biological desulfurization apparatus for biogas. The apparatus comprises a reaction tank, a carrier-packed layer in the reaction tank for adhesion of microorganisms.

Hence, an improved method of converting carbon dioxide and/or carbon mono oxide in a gas and hydrogen in a gas to methane would be advantageous, and in particular, a more efficient method of converting carbon dioxide and/or carbon monoxide and hydrogen in gasses into methane and water would be advantageous.

### Summary of the invention

Thus, an object of the present invention relates to providing an improved method and apparatus for converting carbon dioxide and/or carbon monoxide and hydrogen in a gas to methane and water. The carbon dioxide and/or carbon monoxide is typically provided from one gas source, while hydrogen is provided from another gas source. With the present invention, it will for example be possible increase the conversion of carbon dioxide and/or carbon monoxide and hydrogen in a gas to methane and water because the method and apparatus of the invention comprises an efficient cleaning and decompression system. Further, with the present invention it will be possible to increase the amount of green energy harvested from anaerobic digestion of an organic substrate.

In particular, it is an object of the present invention to convert carbon dioxide and/or carbon monoxide from a gas (for example a gas from an anaerobic digester) and hydrogen generated from electrolysis of water (or similar technologies powered by renewable energy sources such as wind power, solar power and hydropower) to a renewable energy. The renewable energy (methane) can be stored either as a compressed or liquefied gas. The conversion of electric power from renewable energy to an energy form that can be stored is also known as Power-to-X. Therefore, the present invention can also be described as a biological Power-to-X method and apparatus.

Furthermore, it is an object of the present invention to provide a method and apparatus for converting carbon dioxide and/or carbon monoxide in a gas and hydrogen from another gas to methane and water, where the method and apparatus have an improved efficiency in converting carbon dioxide and/or carbon monoxide and hydrogen to methane and water than what is known in prior art. The method and apparatus according to the present invention uses a reactor comprising a packaging material selected from the group of polyethylene, polypropylene, polystyrene and acrylonitrile butadiene styrene (ABS plastic) that makes it possible to obtain a more efficient conversion of carbon dioxide and/or carbon monoxide and hydrogen in a gas to methane and water. By using a packaging material as disclosed above, it is possible to obtain an improved distribution of the gas comprising carbon dioxide and/or carbon monoxide and hydrogen in the reactor. Furthermore, the growth conditions for the microorganisms that converts carbon dioxide and hydrogen and/or carbon monoxide and hydrogen to methane and water is improved. Microorganisms can better grow on the surface of the packaging material than directly in a liquid. The packaging material will provide a large surface for the microorganisms to grow on. Further, the packaging material in the present invention is capable of being detached from the reactor. Hereby, the packaging material can more easily be cleaned and decompressed to remove build-up bio sludge on the packaging material without being removed from the reactor. Hereby, channeling of the gas is avoided, since compression and build-up of bio sludge on the packaging material may lead to formation of channels in the packaging material so that the gas will not be efficiently distributed inside the reactor. Hence, the cleaning and decompression system of the invention results in an improved conversion of carbon dioxide and/or carbon monoxide and hydrogen in the gas to methane and water.

It is a further object of the present invention to provide an alternative to prior art.

Thus, one aspect of the invention relates to a method of converting carbon monooxide and/or carbon dioxide and hydrogen in a gas to methane, comprising the following steps:
a) providing a gas comprising carbon monooxide and/or carbon dioxide;
b) providing a gas comprising hydrogen;
c) leading the gas comprising carbon monooxide and/or carbon dioxide and the gas comprising hydrogen to a reactor, wherein the reactor comprises a packaging material selected from the group consisting of polyethylene (PE), polypropylen (PP), polystyren (PS) and acrylonitrile butadiene styrene (ABS) and the reactor comprises microorganisms capable of converting carbon monooxide and/or carbon dioxide and hydrogen to methane, and wherein the packaging material is capable of being detached from the reactor;
d) providing a liquid and supplying the liquid to the reactor above the packaging material;
e) leading the gas comprising carbon monooxide and/or carbon dioxide and the gas comprising hydrogen through the packaging material comprising the liquid and microorganisms to convert carbon monooxide and/or carbon dioxide and hydrogen to methane and water;
f) leading the gas where carbon monooxide and/or carbon dioxide and hydrogen is converted to methane to an outlet, and
g) cleaning and decompression of the packaging material (2) by filling the reactor with liquid and blowing pressurized air into the reactor (1) below the packaging material (2).

Another aspect of the present invention relates to an apparatus for converting carbon monooxide and/or carbon dioxide and hydrogen in a gas to methane, said apparatus comprises:
i) a reactor (1) comprising a packaging material (2), wherein the packaging material is selected from the group consisting of polyethylene (PE), polypropylene (PP), polystyrene (PS) and acrylonitrile butadiene styrene (ABS), and wherein microorganisms capable of converting carbon monooxide and/or carbon dioxide and hydrogen to methane are growing on the surface of the packaging material, and wherein the packaging material (2) is capable of being detached from the reactor (1);
ii) an inlet (3) for supplying a gas comprising carbon monooxide and/or carbon dioxide to the reactor (1);
iii) an inlet (4) for supplying a gas comprising hydrogen to the reactor (1);
iv) an inlet (5) for supplying liquid to the reactor (1);
v) means (5A) for circulating liquid from the lower part of the reactor (1) to the upper part of the reactor (1) above the packaging material;
vi) an outlet (6) for outletting gas where carbon monooxide and/or carbon dioxide and hydrogen have been converted to methane;
vii)an outlet (7) for outletting liquid from the reactor (1); and
viii) an inlet (9) for blowing pressurized air into the reactor (1), wherein the inlet (9) is located below the packaging material.

### Brief description of the figures

The present invention and in particular preferred embodiments thereof will now be described in more detail with regard to the accompanying figure. The figure shows one way of implementing the present invention and is not to be construed as being limiting to other possible embodiments falling within the scope of the attached claim set.

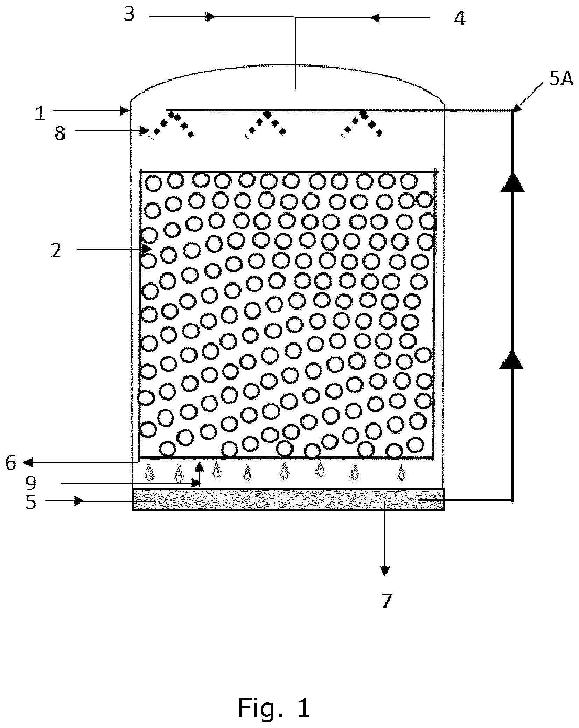

Figure 1 is a schematically illustration of an apparatus for use in converting carbon monooxide and/or carbon dioxide in a gas to methane according to an embodiment of the invention.

The present invention will now be described in more detail in the following.

### Detailed description of the invention

### The method according to the present invention:

In a first aspect, the present invention relates to a method of converting carbon monooxide and/or carbon dioxide and hydrogen in a gas to methane, comprising the following steps:
a) providing a gas comprising carbon monooxide and/or carbon dioxide;
b) providing a gas comprising hydrogen;
c) leading the gas comprising carbon monooxide and/or carbon dioxide and the gas comprising hydrogen to a reactor, wherein the reactor comprises a packaging material selected from the group consisting of polyethylene (PE), polypropylen (PP), polystyren (PS) and acrylonitrile butadiene styrene (ABS) and the reactor comprises microorganisms capable of converting carbon monooxide and/or carbon dioxide and hydrogen to methane, and wherein the packaging material is capable of being detached from the reactor;
d) providing a liquid and supplying the liquid to the reactor above the packaging material;
e) leading the gas comprising carbon monooxide and/or carbon dioxide and the gas comprising hydrogen through the packaging material comprising the liquid and microorganisms to convert carbon monooxide and/or carbon dioxide and hydrogen to methane and water;
f) leading the gas where carbon monooxide and/or carbon dioxide and hydrogen is converted to methane to an outlet, and
g) cleaning and decompression of the packaging material by filling the reactor with liquid and blowing pressurized air into the reactor below the packaging material.

The present invention relates in an aspect to a method where carbon monooxide and/or carbon dioxide in combination with hydrogen in a gas is converted to methane according to claim 1.

The gas comprising carbon monooxide and/or carbon dioxide may be any gas comprising carbon monooxide and/or carbon dioxide. However, preferably, the gas comprising carbon monooxide and/or carbon dioxide is an anaerobic gas.

In an embodiment, the gas comprising carbon monooxide and/or carbon dioxide is selected from the group consisting of biogas, flue gas and syngas. In a preferred embodiment, the gas comprising carbon monooxide and/or carbon dioxide is a biogas produced by an anaerobic digester.

Biogas is a gas produced as a waste product/by-product from an anaerobic digester. The anaerobic digester is treating industrial and agricultural organic waste streams in an oxygen free environment. The organic material treated in the anaerobic digester may be manure from farm animals, food processing plants, breweries, palm oil mills, starch factories, ethanol plants, paper mills, municipal sewage treatment plants and sorted household. The biogas obtained after treatment in the anaerobic digester is anaerobic, i.e. containing no oxygen. Another example of a biogas is landfill gas that is a biogas from a landfill with organic deposits.

Flue gas is the gas discharged to the atmosphere via a flue, which is a pipe or channel for conveying exhaust gases from a fireplace, oven, furnace, boiler or steam generator. Often, the flue gas refers to the combustion exhaust gas produced at power plants based on coal or biomass and incinerators. For example, flue gas can come from thermal power plants, waste incineration plants or cement factories. The composition of a flue gas depends on what is being burned, but will usually comprise nitrogen, carbon dioxide and water vapour. It may also comprise oxygen and small amounts of pollutants such as particulate matter (e.g. soot), carbon monoxide, nitrogen oxide and sulphur oxide. Carbon dioxide may be present in the flue gas in an amount of 10-25 volume percent or more of the flue gas.

Syngas, may also be referred to as synthesis gas, is a fuel gas formed from pyrolysis and gasification of organic materials such as coal, biomass, and in principle any hydrocarbon feedstock, for example wood and straw. The composition of the syngas depends on which organic material is used, and will typically comprise hydrogen, carbon monoxide, carbon dioxide and methane.

The gas comprising hydrogen may be any gas comprising hydrogen. For example, the hydrogen gas may be obtained by electrolysis of water or similar technologies. Hydrogen gas is supplied to the reactor in an amount leading to at least a part of carbon dioxide and/or carbon monoxide in a gas is converted to methane and dihydrogenmonoxide (water). The amount of hydrogen added will also depend on the amount of carbon monoxide and/or carbon dioxide in the gas led to the reactor. Hence, the amount of hydrogen supplied is not an essential parameter of the present invention and is dependent on the amount of carbon monoxide and/or carbon dioxide that is desired to be converted.

The hydrogen gas may either be added directly to the gas comprising carbon monoxide and/or carbon dioxide before the gas is lead to the reactor, such that a mixture of gas comprising carbon monoxide and/or carbon dioxide and hydrogen is led to the reactor. Alternatively, the gas comprising carbon monoxide and/or carbon dioxide and the gas comprising hydrogen are led to the reactor separately. In a preferred embodiment of the invention, the gas comprising carbon monoxide and/or carbon dioxide and the gas comprising hydrogen is mixed before introduced into the reactor.

In an embodiment of the present invention, the amount of gas comprising hydrogen and the gas comprising carbon monoxide and/or carbon dioxide introduced to the reactor is controlled. This may for example be by using a frequency regulated blower and for example adjusting the injection of the gas comprising hydrogen to the amount of carbon monoxide and/or carbon dioxide introduced, such that only the amount of hydrogen needed for conversion of carbon monoxide and/or carbon dioxide to methane and water is introduced.

The reactor used in the method according to the present invention comprises a packaging material and may therefore be referred to as a biotrickling filter or biotrickling reactor.

It is an important aspect of the present invention that the packaging material in the reactor is made of a material selected from the group consisting of polyethylene (PE), polypropylene (PP), polystyrene (PS) and acrylonitrile butadiene styrene (ABS). Polyurethane is another material known to be used as packaging materials. However, polyurethane is not suitable since polyurethane is too soft when exposed to pressure and/or higher temperatures. A packaging material selected from polyethylene (PE), polypropylene (PP), polystyrene (PS) and acrylonitrile butadiene styreneis more resistant against the pressure from the weight of the packaging material itself than for example polyuretane. In commercial full-scale plants, the packaging material may be stacked up to a height of several meters. Even though the packaging material is selected from polyethylene (PE), polypropylene (PP), polystyrene (PS) and acrylonitrile butadiene styrene, there is a risk that the packaging material will be compressed by the weight of the packing material itself and liquid when said liquid is supplied to the reactor (sprinkled over the packaging material), and when bio-sludge is formed on the packaging material. Therefore, it is important that the packaging material occasionally is cleaned and decompressed according to the present invention. The cleaning removes build-up bio-sludge and decompress the packaging material if it has been compressed.

Acrylonitrile butadiene styrene (ABS) is strongest followed by polystyrene, then polypropylene and then polyethylene. By using a packaging material according to the invention in combination with the cleaning and decompression system according to the invention, it is possible to stack the packaging material up to a height of 10-12 meters while having a temperature in the reactor of up to 70°C. If a packaging material having less strength is chosen, the packaging material will be compressed and the surface area where the microorganisms can grow is decreased. Eventually, the compression of the packaging material can be so great that gas and liquid cannot pass through the filter. Hence, if the packaging material is compressed, it is important to rapidly and efficiently clean and decompress the packaging material. Removal of the packaging material outside the reactor for cleaning is time-consuming and will stop the methanation process, i.e. the process of converting carbon dioxide and/or carbon monoxide in a gas to methane and water. With the construction of the apparatus according to the present invention and the method of the present invention, it is possible to rapidly and efficiently clean and decompress the packaging inside the reactor. Hereby, an improved method and apparatus of converting carbon dioxide and/or carbon monoxide in a gas to methane and water is obtained.

In addition, the use of a packaging material in the reactor instead of for example bubbling the gasses through a liquid as disclosed in prior art has several advantages. One advantage is that that there will be an improved growth of microorganisms, since the microorganisms grows better on the surface of the packaging material than directly in a liquid. When using a packaging material there will be a larger surface area where the microorganisms can grow. The liquid supplied over the packaging material will surround the large surface area of the packaging material as a thin film. The microorganisms will grow on the surface of the packaging material in those thin films. Hence, it will be easier for the microorganisms to get in contact with the hydrogen and convert carbon dioxide and/or carbon mono oxide in a gas to methane and water. Further, hydrogen is poorly soluble in water and therefore a method using a reactor comprising a packaging material and only a small amount of liquid supplied over the packaging material will improve the solubilisation and distribution of the hydrogen gas in the reactor. However, there is also a risk that the packaging material is compressed by the weight of the packing material itself and the weight of the water supplied to the reactor and the build-up sludge. However, with the method and apparatus of the present invention, the problems of compressed packaging material and sludge build up on the packaging material is overcome by having a cleaning and decompression system that rapidly and efficiently can clean and decompress the packaging material without removing it from the reactor.

Hence, in an aspect of the present invention, the packaging material is capable of being detached from the reactor. The packaging material can be detached from both the bottom and the sidewalls of the reactor. This makes it possible to obtain an efficient cleaning of the reactor.

It is necessary to clean the reactor comprising the packaging material occasionally to avoid compression and clogging of the packaging material. If the packaging material is compressed or clogged, the passage of gasses and liquid through the packaging material will over time be reduced or completely blocked. This will decrease and eventually stop the convention of carbon monoxide and/or carbon dioxide and hydrogen to methane. The cleaning can be made with a system where the reactor is filled with liquid, for example water or effluent from a digester, followed by blowing pressurized air into the lower part of the reactor. The pressurized air is blown into the reactor below the packaging material. Hereby, the packaging material is lifted from the bottom of the tank and flows freely in the liquid and is being washed. The cleaning will counteract compression of the packaging material and also remove built-up bio sludge without the need of emptying the reactor for the packaging media. Hence, if the packaging material can be detached from the reactor, a more efficient cleaning and decompression of the reactor is possible.

The liquid applied during the cleaning step g) in the method according to the invention may for example be selected from the group consisting of water and effluent from an anaerobic digester. The liquid applied during the cleaning step may be the same liquid as supplied to the reactor in step d) in the method according to the invention or may be a different liquid. Preferably, the liquid supplied in step d) and in step g) is the same liquid.

The terms "bio-sludge" and "sludge" refers in the context of the present invention to build-up deposits of solid matter.

In an embodiment of the invention, the pressurized air is blown into the reactor from an inlet that supplies pressurized air. The inlet for pressurized air is located below the packaging material. The inlet may in an embodiment comprise two or more inlets for blowing pressurized air.

The inlets for supplying pressurized air are preferably two or more inlets located in a distance from each other. This construction of multiple inlets for pressurized air results in that pressurized air is supplied to the reactor over the entire cross section of the reactor, such that the packaging material efficiently can be lifted up and cleaned. Preferably, the inlets are located in a distance from each other to provide one inlet per up to 10 m² of the cross section of the reactor, preferably in a distance from each other to provide one inlet per up to 7 m² of the cross section of the reactor, such as in a distance from each other to provide one inlet per up to 5 m² of the cross section of the reactor, and more preferably in a distance from each other to provide one inlet per up to 3 m² of the cross section of the reactor, most preferably in a distance from each other to provide one inlet per up to 2 m² of the cross section of the reactor

The microorganisms used in the present invention is any microorganism capable of converting carbon monooxide and hydrogen and/or carbon dioxide and hydrogen to methane and dihydrogenmonoxide (water). Preferably, the microorganism are methanogens, i.e. methanogenic microorganisms. The microorganisms are growing on the surface of the packaging material

The microorganisms convert carbon dioxide and/or carbon monooxide into methane and water by reaction with hydrogen:
- CO₂ + 4H₂ → CH₄ + 2 H₂O + heat
- CO + 3 H₂ → CH₄ + H₂O + heat

The amount of hydrogen is dependent of how much carbon dioxide is present and how much carbon monoxide is present. For example, if the gas only comprises carbon dioxide and not carbon monoxide, hydrogen should preferably be supplied in the amount of 4 mole hydrogen for every mole of carbon dioxide.

The methanogenic microorganisms, or autotrophic methanogenic microorganisms may be anaerobic archaes. The methanogenic microorganisms may be provided as one strain or a combination of two or more strains. The specific strain of methanogenic microorganism used in the present invention is not relevant and any methanogenic microorganism may be used. Hence, the present invention is not limited to the specific strain of microorganisms capable of converting carbon dioxide and/or carbon monoxide to methane. However, examples of methanogenic microorganism suitable in the present invention are *Methanobacterium,*

*Methanobrevibacter, Methanothermobacter, Methanococcus, Methanosarcina, Methanopyrus* or mixtures thereof.

Examples of specific strains suitable for use is *Methanobacterium thermoautotrophicum, Methanobacterium omelianskii, Methanobacterium formicicum, Methanococcus vannielii, Methanococcus barkerii* and *Methanococcus thermolithotrophicus.*

The microorganisms used in the present invention for converting carbon monooxide and/or carbon dioxide and hydrogen to methane and water are naturally occurring in effluent from an anaerobic digester. Therefore, the effluent may be used for inoculation of the process in the reactor. When the process has started up, the microorganisms capable of converting carbon monooxide and/or carbon dioxide and hydrogen to methane will multiply, and it is not necessary to provide more anaerobic digester effluent unless the reactor has been out of operation for a longer period of time.

The effluent from an anaerobic digester may be obtained from digesting of any organic material, such as for example digestion of manure from farm animals, food processing plants, breweries, palm oil mills, starch factories, ethanol plants, paper mills, municipal sewage treatment plants and sorted household. The composition of the effluent from an anaerobic digester will depend on the organic material/substrate that is being degassed in the biogas reactor.

Further, the microorganisms require nutrients such as nitrogen (N), phosphorus (P) and potassium (K). The process produces besides from methane and heat also water. Despite that draining of excess water from the reactor is necessary, liquid is typically added to the reactor to provide nutrients for the process.

In an embodiment of the invention, the liquid is selected from the group of water and effluent from an anaerobic digester. If water is used as the liquid source in step d) of the method of the present invention, a fertilizer is supplied to the water or directly to the reactor. The fertilizer may be a NPK fertilizer. If effluent from an aerobic digester is used as liquid source, some or all of the nutrients are typically present in the effluent and the consumption of NPK fertilizer may be limited or avoided.

It is also important to keep pH in the range of 6 to 8 as this is the pH optimum of the microorganisms. Therefore, a suitable base is added to the reactor to maintain the pH level. Alternatively, addition of effluent from an anaerobic digester also function as a pH adjustment. The present invention is not limited to the type of base added and it may be any base.

It is important to supply a liquid over the packaging material in order to provide moisture and nutrients for the microorganisms to grow and to provide cooling.

The liquid may either be supplied to the reactor in step d) continuously or periodically.

If the liquid in step d) is supplied periodically, it may be periodically sprinkling of the liquid or it may be periodically flooding of the packaging material followed by draining of the liquid.

However, in a preferred embodiment supplying of the liquid to the reactor in step d) is a continuously sprinkling. The continuously sprinkling of liquid is preferably with a flowrate of the liquid in the range of 0.5 to 2.5 m³/m² of the area of the cross section of the reactor. The flowrate of liquid should not be lower than 0.5 m³/m² since a flow rate below 0.5 m³/m² results in that the temperature in the reactor increases and becomes higher than the optimal for the microorganisms to grow. The liquid functions as a cooling medium. Further, if the flowrate is below 0.5 m³/m² bio sludge is formed and accumulated in the packaging material such that efficiency is decreased.

The flowrate should not exceed 2.5 m³/m² of the area of the cross section of the reactor, since a higher flowrate will result in stressing of the microorganisms growing on the surface of the packaging material (the biofilm). Further, a flowrate above 2.5 m³/m² result in that the biofilm (including the microorganisms) is flushed of the packaging material such that the carbon dioxide and/or carbon monoxide cannot be converted to methane. Preferably, the flowrate should be in the range of 1.0 to 2.0 m³/m² for the method of converting the carbon dioxide and/or carbon monoxide and hydrogen to methane to be efficient, and most preferably the flowrate should be in the range of 1.2 to 1.8 m³/m². In an example, the flow rate is about 1.5 m³/m2.

The liquid supplied to the reactor in step d) above the packaging material may in an embodiment be liquid recirculated from the sump in the bottom of the reactor. If such sump is recirculated and supplied to the upper part of the reactor, it may be cooled before supplied to the reactor. Such cooling may be by any known device suitable for cooling.

The gas comprising carbon monoxide and/or carbon dioxide and the gas comprising hydrogen is led through the packaging material where microorganisms are growing. When the gasses are led through the packaging material, the microorganisms will convert the carbon monoxide and/or carbon dioxide and hydrogen to methane and water.

The gasses can for example be applied at the top of the reactor, above the packaging material, and be lead co-currently through the packaging material. In this situation, the gas where carbon monooxide and/or carbon dioxide and hydrogen have been converted to methane is let out from an outlet located in the lower part of the reactor.

The gasses may also be applied to the lower part of the reactor, below the packaging material, and be led counter-currently through the packaging material. In this situation, the gas where carbon monooxide and/or carbon dioxide and hydrogen have been converted to methane is let out from an outlet located in the upper part of the reactor.

Whether the gasses are driven co-current or counter-current is depending on the design of the reactor.

The excess water created in the reactor can be outlet through an outlet placed in the lower part of the reactor, preferably below the packaging material.

The method according to the present invention may also comprise a cooling system to discharge heat developed in the reactor during the conversion of carbon monooxide and/or carbon dioxide to methane. The liquid applied to the reactor will for example cool the reactor.

In an embodiment of the invention, the temperature in the reactor should be in the range of 40°C to 65°C. The temperature in the reactor should maintained at this temperature by providing cooling. Any suitable cooling system could be used, and the invention is not limited to the use of a particular cooling system. Typically, cooling provided to the reactor comes from the liquid supplied to the reactor. For example, if the sump from the bottom of the reactor is recirculated to the reactor, it passes means for cooling before supplied to reactor. The means for cooling is typically a heat exchanger. The temperature in the reactor should be in the range of 40°C to 65°C because the microorganisms will grow in this temperature range. Preferably, the temperature in the reactor is 45°C to 60°C that gives optimal conditions for the microorganisms to grow.

The method according to the present invention may also comprise a heating system to provide heat to the reactor. When the method is starting up, effluent from an anaerobic digester is supplied to the reactor. The effluent comprises microorganisms than can convert carbon monoxide and/or carbon dioxide and hydrogen to methane and water. However, for the microorganisms to have optimal growth conditions, heat needs to be supplied to the reactor to increase the temperature to 40°C to 65°C. Any suitable heating system could be used, and the invention is not limited to the use of a particular heating system. As an example, the heating may be by using heat exchangers. After start-up, the exothermic process will generate heat and instead of heating, cooling is needed.

In countries with tropical climate there is no need for insulation of the reactors, but in moderate and colder climates the reactors may be insulated.

In a preferred embodiment of the method according to the invention, the cooling and heating system is a combined cooling-heating system.

The method according to the present invention also comprises pH stabilisation of the liquid to maintain pH at 6.0 to 8.0. The optimal pH for the microorganisms to grow is in the range of 6.0 to 8.0. Any pH adjusting agent may be used for the pH stabilisation. However, in an embodiment of the invention, decanted and degassed effluent from an anaerobic digester is supplied for stabilising the pH.

The method is carried out with a positive pressure, below 200 mbar, such that vacuum in the reactor is avoided. Hence, in an embodiment of the method according to the present invention, the pressure in the reactor is 200 mbar or below.

Hence, the method according to the present invention makes it possible to efficiently and biologically to convert carbon monoxide and/or carbon dioxide and hydrogen, for example obtained by electrolysis of water, to methane. The method of the present invention has the advantage of being carried out at ordinary temperatures (40-65°C) and normally without pressure (below 200 mbar). However, the method is also capable of being performed under pressure, and for example under pressure in the range of 2-10 bar. The method of the present invention should therefore not be limited to any pressure. Catalytic processes uses temperatures of 250-900°C and a pressure of 10-30 bar. Furthermore, the method according to the present invention is not dependent of hydrogen sulphide is removed from the gas comprising carbon monoxide and/or carbon dioxide, for example biogas, but can be used on raw biogas that typically comprises 3000-5000 ppm hydrogen sulphide. However, the method does not require hydrogen sulphide present. Therefore, hydrogen sulphide may optionally be removed from the gas with carbon monoxide and/or carbon dioxide before the method of the invention is carried out.

The method according to the present invention makes it possible to increase the content of methane in a gas, such as biogas, flue gas and syngas. In for example a biogas, the content of methane in the gas is increased from typically 50-60% to more than 95%. Hence, the utilisation of the biogas obtained from the anaerobic digester is increased significantly.

Liquid from the reactor may be outlet from the reactor and in an embodiment, the liquid outlet from the reactor is recirculated to the reactor and supplied above the packaging material in step d) or the cleaning in step g).

In a preferred embodiment, the method according to the invention is anaerobic, i.e. the method is carried out under anaerobic conditions. The optimal growth of microorganisms capable of converting carbon monooxide and/or carbon dioxide and hydrogen to methane are at anaerobic conditions.

### Apparatus according to the invention:

Reference is made in the following to figure 1 by use inter alia of the reference numbers presented in that figure. Figure 1 schematically illustrates an apparatus for converting carbon monooxide and/or carbon dioxide in a gas and hydrogen to methane according to the present invention.

In a second aspect, the present invention relates to an apparatus for converting carbon monooxide and/or carbon dioxide in a gas and hydrogen to methane, said apparatus comprises:
i) a reactor (1) comprising a packaging material (2), wherein the packaging material is selected from the group consisting of polyethylene (PE), polypropylene (PP), polystyrene (PS) and acrylonitrile butadiene styrene (ABS), and wherein microorganisms capable of converting carbon monooxide and/or carbon dioxide and hydrogen to methane are growing on the surface of the packaging material, and wherein the packaging material (2) is capable of being detached from the reactor (1);
ii) an inlet (3) for supplying a gas comprising carbon monooxide and/or carbon dioxide to the reactor (1);
iii) an inlet (4) for supplying a gas comprising hydrogen to the reactor (1);
iv) an inlet (5) for supplying liquid to the reactor (1);
v) means (5A) for circulating liquid from the lower part of the reactor (1) to the upper part of the reactor (1) above the packaging material;
vi) an outlet (6) for outletting gas where carbon monooxide and/or carbon dioxide and hydrogen have been converted to methane;
vii) an outlet (7) for outletting liquid from the reactor (1), and
viii) an inlet (9) for blowing pressurized air into the reactor (1), wherein the inlet (9) is located below the packaging material (2).

As illustrated in figure 1, the apparatus comprises a reactor (1) comprising a packaging material (2). The packaging material is selected from the group consisting of polyethylene (PE), polypropylene (PP), polystyrene (PS) and acrylonitrile butadiene styrene (ABS). The apparatus is furthermore configured to comprise microorganisms growing on the surface of the packaging material and said microorganisms are capable of converting carbon monooxide and/or carbon dioxide and hydrogen to methane. The reactor may be made of a material resistant to various components contained in the reactor, and is for example made from fibre-reinforced plastic, stainless steel or coated carbon steel.

In an aspect of the apparatus of the invention, the packaging material (2) is capable of being detached from the reactor (1). When the packaging material are detachable from the reactor, it is possible to lift it up when filling the tank with liquid and blowing pressured air into the reactor by inlet for blowing presurized air into the reactor. Hereby, the reactor can be cleaned from accumulated bio sludge and prevent clogging and compression of the packaging material. If the packaging material is clogged or compressed, the flow of gasses and liquid through the packaging material is decreased.

The inlet (9) for blowing pressurized air preferably comprises two or more inlets for blowing pressurized air.

The inlets for supplying pressurized air (9) are preferably two or more inlets located in a distance from each other. This construction of multiple inlets for pressurized air results in that pressurized air is supplied to the reactor over the entire cross section of the reactor, such that the packaging material efficiently can be lifted up and cleaned. Preferably, the inlets are located in a distance from each other to provide one inlet per up to 10 m² of the cross section of the reactor, preferably in a distance from each other to provide one inlet per up to 7 m² of the cross section of the reactor, such as in a distance from each other to provide one inlet per up to 5 m² of the cross section of the reactor, and more preferably in a distance of from each other to provide one inlet per up to 3 m² of the cross section of the reactor, most preferably in a distance from each other to provide one inlet per up to 2 m² of the cross section of the reactor

The apparatus comprises an inlet (3) for supplying a gas comprising carbon mono oxide and/or carbon dioxide and an inlet (4) for supplying a gas comprising hydrogen to the reactor (1). In figure 1, the inlets (3) and (4) for supplying gasses are located in the upper part of the reactor (1) above the packaging material (2). In this embodiment of the invention, the gasses will be led co-current through the packaging material, i.e. in the same direction as the liquid flows and the outlet (6) for outletting gas where carbon monoxide and/or carbon dioxide and hydrogen is converted to methane is located in the lower part of the reactor (1). However, inlets (3) and (4) for supplying gasses may also be located in the lower part of the reactor (1), below the packaging material (2). In such embodiment of the invention, the gasses will be led counter-current through the packaging material (2), i.e. in the opposite direction than the flow of the liquid. When the gas is led counter-current, the outlet (6) is located in the upper part of the reactor (1), above the packaging material (2).

In figure 1, the gas comprising carbon mono oxide and/or carbon dioxide and the gas comprising hydrogen is mixed before supplied to the reactor. However, in an alternative embodiment of the invention, the gas comprising carbon mono oxide and/or carbon dioxide and the gas comprising hydrogen may be supplied to the reactor separately.

The gas inlets may be provided with a blower (not shown) to lead the gas into the reactor (1). If the gas has a pressure being higher than the pressure inside the reactor, the blower may be replaced by a control valve (not shown) for controlling the flow of gas into the reactor. A combination of a blower and control valve may be preferred also in cases where the gasses need to be led into the reactor such that the amount of gas comprising carbon monoxide and carbon dioxide and the gas comprising hydrogen can be controlled. Further, the means as disclosed above as to regulation of flow may also be applied.

As presented herein, the invention also comprising microoganisms growing on the surface of the packaging material (2) inside the reactor (1). The microorganisms are any microorganism capable of converting carbon monoxide and/or carbon dioxide and hydrogen to methane and water. The microorganisms are as disclosed earlier preferably methanogenic microorganisms and may for example be any of the previous described microorganisms mentioned under the method of the invention.

The apparatus further comprises an inlet (5) for supplying a liquid to the reactor. The inlet (5) may be one or more inlets. The inlet (5) for supplying liquid is preferably supplied to the lower part of the reactor (1). The apparatus further comprises means (5A) for circulating liquid from the lower part of the reactor (1) to the upper part of the reactor (1) above the packaging material (2) and the flow of the liquid will lead it through the packaging material. This provides moisture for the growth of microorganisms. In the embodiment of the invention shown in figure 1, the inlet (5) is located in the lower part of the reactor (1) and is mixed with liquid already led through the packaging material, said mixture of liquid is recirculated by means for recirculation (5A) and supplied to the reactor in the upper part of the reactor (1) above the packaging material (2). Hence, the reactor comprises means for circulation (5A) of liquid from the lower part of the reactor (1) to the upper part of the reactor (1). The liquid recirculated will typically pass means for cooling, for example a heat exchanger, to cool the liquid before supplied to the reactor. Hence, in an embodiment the apparatus also comprises means for cooling the liquid supplied to the reactor.

In another embodiment (not shown), the inlet (5) of liquid can be located as a valve connected to the upper part of the reactor (1).

In a further embodiment of the invention, the reactor (1) comprises sprinkling means (8) for sprinkling the liquid into the reactor. The sprinkling means are preferably connected to the means for recirculation (5A) liquid. Further, the sprinkling means (8) may comprise controlling means for controlling the flowrate of the liquid sprinkled into the reactor. The flowrate of the liquid supplied to the reactor is preferably in the range of 0.5 to 2.5 m³/m² of the area of the cross section of the reactor, more preferably 1.0 to 2.0 m³/m².

The apparatus also comprises an outlet (6) for outletting the gas where carbon monoxide and/or carbon dioxide and hydrogen have been converted to methane. This gas may also be referred to as a methane gas or biomethane gas. The gas comprising carbon monoxide and/or carbon dioxide and hydrogen is converted to methane while inside the reactor and the resulting biomethane gas can be outlet from the reactor from the outlet (6).

In addition, the apparatus preferably comprises an outlet (7) for outletting liquid from the reactor (1). Water is formed when carbon monoxide and hydrogen and/or carbon dioxide and hydrogen is/are converted to methane. Hence, the water content in the reactor may increase and it may be needed to discharge some liquid.

When the apparatus for converting carbon monooxide and/or carbon dioxide and hydrogen in a gas to methane starts up, microorganisms are introduced to the reactor, for example by supplying an effluent from an anaerobic digester that comprises methanogenic microorganisms. The temperature in the reactor is increased to optimal temperature for growth of microorganisms, i.e. by heating such that the temperature in the reactor is in the range of 40-70°C. When the apparatus is ready for converting carbon monooxide and/or carbon dioxide and hydrogen in a gas to methane, heating is no longer necessary, since the conversion will create heat. Instead, it will now be necessary to provide cooling to the reactor to keep the temperature in the range of 40°C to 70°C.

In an embodiment of the invention, the reactor (1) comprises a cooling system to discharge heat developed in the reactor during the conversion of carbon monooxide and/or carbon dioxide and hydrogen to methane.

In another embodiment of the invention, the reactor (1) comprises a heating system to provide heat to the reactor during start-up. The cooling and heating system may be a combined cooling-heating system.

The cooling and/or heating system is/are provided to maintain the temperature in the reactor in the range of 40°C to 70°C. This is the optimal temperature for growth of microorganisms.

It should be noted that embodiments and features described in the context of one of the aspects of the present invention also apply to the other aspects of the invention. For example, embodiments described in connection with the method of the invention also apply to the apparatus of the invention.

The invention will now be described in further details in the following non-limiting examples.

### Examples

### Example 1:

An example was made to show compression of 1) polyurethane foam, 2) polyethylene and 3) polypropylene as packaging materials.

Samples of each packaging material being 46 cm high were placed in a pipe having an internal diameter of 100 mm. The temperature was 30°C. A load of 1.9 kg was added to each pipe simulating a weight of 240 kg/m2 surface. This is to simulate the weight of liquid and bio-film on the packaging material used in the method and apparatus according to the present invention. A load of 240 kg/m2 is considered a very moderate load. After the load was added to each pipe, the height of the packaging material were measured again to find out how much the packaging material had been compressed.

In table 1 below, the height of the different packaging materials is listed before and after the weight load.

**Table 1:**

| | Polyurethane foam | Polyethylene | Polypropylene |
|---|---|---|---|
| Height before load (cm) | 46 | 46 | 46 |
| Height after load (cm) | 33 | 46 | 46 |

Hence, if polyurethane is used as the packaging material the packaging material is compressed by approximately 28% despite this very moderate load. Polyurethane foam is therefore unsuitable as packaging material in the method and apparatus according to the present invention. On the contrary, there is no compression of the polyethylene or polypropylene when a load of 240 kg/m2 is applied.

### Example 2:

An example was made using a higher load to test the difference in compression of 1) polyethylene and 2) polypropylene as packaging materials.

Samples of each packaging material were placed in a pipe having an internal diameter of 100 mm. The temperature was 32°C. A load of 48 kg was added to each pipe simulating a weight of 6000 kg/m2 surface which is the expected maximum load on the lower part of the packaging material used in the present invention with a height of the packaging material of 10 m. The main contribution to the high load is wet bio sludge build up on the surface of the packaging material.

In table 2 below, the height of the different packaging materials is listed before and after the weight load.

**Table 2:**

| | Polyethylene | Polypropylene |
|---|---|---|
| Height before load (mm) | 453 | 455 |
| Height after load (mm) | 434 | 448 |

Hence, with a load of 48 kg, the packaging material of polyethylene were compressed 19 mm, which correspond to 4.19%. On the contrary, the packaging material made of polypropylene only compressed 7 mm, corresponding to 1.54% with this very high load.

Hence, the test results shows that both the use of polypropylene and polyethylene as the packaging material resulted in compression of the packaging material. The highest compression was with the use of polyethylene as packaging material. Hence, the packaging material are compressed during the methanation process. To avoid decreased efficiency by clogging of the packaging material, efficient cleaning and decompression according to the invention is necessary.

## Claims

1. A method of converting carbon monooxide and/or carbon dioxide and hydrogen in a gas to methane, comprising the following steps:
a) providing a gas comprising carbon monooxide and/or carbon dioxide;
b) providing a gas comprising hydrogen;
c) leading the gas comprising carbon monooxide and/or carbon dioxide and the gas comprising hydrogen to a reactor, wherein the reactor comprises a packaging material selected from the group consisting of polyethylene (PE), polypropylen (PP), polystyren (PS) and acrylonitrile butadiene styrene (ABS) and the reactor comprises microorganisms capable of converting carbon monooxide and/or carbon dioxide and hydrogen to methane, and wherein the packaging material is capable of being detached from the reactor;
d) providing a liquid and supply the liquid to the reactor above the packaging material;
e) leading the gas comprising carbon monooxide and/or carbon dioxide and the gas comprising hydrogen through the packaging material comprising the liquid and microorganisms to convert carbon monooxide and/or carbon dioxide and hydrogen to methane and water;
f) leading the gas where carbon monooxide and/or carbon dioxide and hydrogen is converted to methane to an outlet, and
g) cleaning and decompression of the packaging material by filling the reactor with liquid and blowing pressurized air into the reactor below the packaging material.

2. The method according to claim 1, wherein the microorganisms are methanogenic microorganisms.

3. The method according to any of the claims 1 to 2, wherein the liquid is selected from the group consisting of water and effluent from an anaerobic digester.

4. The method according to any of the claims 1 to 3, wherein supplying of a liquid to the reactor is a continuously sprinkling.

5. The method according to claim 4, wherein the continuous sprinkling is with a flowrate of the liquid in the range of 0.5 to 2.5 m³/m² of the area of the cross section of the reactor.

6. The method according to any of the claims 1 to 5, wherein the gas comprising carbon monooxide and/or carbon dioxide is selected from the group consisting of biogas, flue gas and syngas.

7. The method according to any of the claims 1 to 6, wherein the gas comprising carbon monooxide and/or carbon dioxide is an anaerob gas.

8. The method according to the claims 1 to 7, wherein the temperature in the reactor is in the range of 40°C to 70°C.

9. The method according to claim 8, wherein the method is anaerobic.

10. An apparatus for converting carbon monooxide and/or carbon dioxide and hydrogen in a gas to methane, said apparatus comprises:
i) a reactor (1) comprising a packaging material (2), wherein the packaging material is selected from the group consisting of polyethylene (PE), polypropylene (PP), polystyrene (PS) and acrylonitrile butadiene styrene (ABS), and wherein microorganisms capable of converting carbon monooxide and/or carbon dioxide and hydrogen to methane are growing on the surface of the packaging material, and wherein the packaging material (2) is capable of being detached from the reactor (1);
ii) an inlet (3) for supplying a gas comprising carbon monooxide and/or carbon dioxide to the reactor (1);
iii) an inlet (4) for supplying a gas comprising hydrogen to the reactor (1);
iv) an inlet (5) for supplying liquid to the reactor (1)
v) means (5A) for circulating liquid from the lower part of the reactor (1) to the upper part of the reactor (1) above the packaging material;
vi) an outlet (6) for outletting gas where carbon monooxide and/or carbon dioxide and hydrogen have been converted to methane;
vii) an outlet (7) for outletting liquid from the reactor (1), and
viii) an inlet (9) for blowing pressurized air into the reactor (1), wherein the inlet (9) is located below the packaging material (2).

11. The apparatus according to claim 10, wherein the reactor (1) comprises sprinkling means (8) for sprinkling the liquid above the packaging material.

12. The apparatus according to claim 11, wherein the sprinkling means (8) comprises controlling means for controlling the flowrate of the liquid sprinkled into the reactor to be in the range of 0.5 to 2.5 m³/m² of the area of the cross section of the reactor.

## Patentansprüche

1. Verfahren zum Umwandeln von Kohlenmonoxid und/oder Kohlendioxid und Wasserstoff in einem Gas zu Methan, umfassend die folgenden Schritte:
a) Bereitstellen eines Gases, umfassend Kohlenmonoxid und/oder Kohlendioxid;
b) Bereitstellen eines Gases, umfassend Wasserstoff;
c) Leiten des Gases, umfassend Kohlenmonoxid und/oder Kohlendioxid, und des Gases, umfassend Wasserstoff, zu einem Reaktor, wobei der Reaktor ein Verpackungsmaterial umfasst, das ausgewählt ist aus der Gruppe bestehend aus Polyethylen (PE), Polypropylen (PP), Polystyrol (PS) und Acrylnitril-Butadien-Styrol (ABS), und der Reaktor Mikroorganismen umfasst, die in der Lage sind, Kohlenmonoxid und/oder Kohlendioxid und Wasserstoff zu Methan umzuwandeln, und wobei das Verpackungsmaterial in der Lage ist, vom Reaktor abgenommen zu werden;
d) Bereitstellen einer Flüssigkeit und Zuführen der Flüssigkeit zu dem Reaktor oberhalb des Verpackungsmaterials;
e) Leiten Gases, umfassend Kohlenmonoxid und/oder Kohlendioxid, und des Gases, umfassend Wasserstoff, durch das Verpackungsmaterial, umfassend die Flüssigkeit und Mikroorganismen, zum Umwandeln von Kohlenmonoxid und/oder Kohlendioxid und Wasserstoff zu Methan und Wasser;
f) Leiten des Gases, in dem Kohlenmonoxid und/oder Kohlendioxid und Wasserstoff zu Methan umgewandelt werden, zu einem Auslass, und
g) Reinigen und Dekomprimierung des Verpackungsmaterials durch Befüllen des Reaktors mit Flüssigkeit und Einblasen von Druckluft in den Reaktor unterhalb des Verpackungsmaterials.

2. Verfahren nach Anspruch 1, wobei die Mikroorganismen methanogene Mikroorganismen sind.

3. Verfahren nach einem der Ansprüche 1 bis 2, wobei die Flüssigkeit ausgewählt ist aus der Gruppe bestehend aus Wasser und Abwasser aus einem anaeroben Faulbehälter.

4. Verfahren nach einem der Ansprüche 1 bis 3, wobei ein Zuführen einer Flüssigkeit zum Reaktor durch kontinuierliches Besprühen erfolgt.

5. Verfahren nach Anspruch 4, wobei das kontinuierliche Besprühen mit einer Durchflussrate der Flüssigkeit im Bereich von 0,5 bis 2,5 m³/m² der Querschnittsfläche des Reaktors erfolgt.

6. Verfahren nach einem der Ansprüche 1 bis 5, wobei das Kohlenmonoxid und/oder Kohlendioxid umfassende Gas ausgewählt ist aus der Gruppe bestehend aus Biogas, Rauchgas und Synthesegas.

7. Verfahren nach einem der Ansprüche 1 bis 6, wobei das Kohlenmonoxid und/oder Kohlendioxid umfassende Gas ein anaerobes Gas ist.

8. Verfahren nach den Ansprüchen 1 bis 7, wobei die Temperatur in dem Reaktor im Bereich von 40 °C bis 70 °C liegt.

9. Verfahren nach Anspruch 8, wobei das Verfahren anaerob ist.

10. Vorrichtung zum Umwandeln von Kohlenmonoxid und/oder Kohlendioxid und Wasserstoff in einem Gas zu Methan, umfassend:
i) einen Reaktor (1), umfassend ein Verpackungsmaterial (2), wobei das Verpackungsmaterial ausgewählt ist aus der Gruppe bestehend aus Polyethylen (PE), Polypropylen (PP), Polystyrol (PS) und Acrylnitril-Butadien-Styrol (ABS), und wobei Mikroorganismen, die in er Lage sind, Kohlenmonoxid und/oder Kohlendioxid und Wasserstoff zu Methan umzuwandeln, auf der Oberfläche des Verpackungsmaterials wachsen, und wobei das Verpackungsmaterial (2) in der Lage ist, von dem Reaktor (1) abgenommen zu werden;
ii) einen Einlass (3) zum Zuführen eines Gases, umfassend Kohlenmonoxid und/oder Kohlendioxid zu dem Reaktor (1);
iii) einen Einlass (4) zum Zuführen eines Gases, umfassend Wasserstoff, zu dem Reaktor (1);
iv) einen Einlass (5) zum Zuführen von Flüssigkeit zu dem Reaktor (1);
v) eine Einrichtung (5A) zum Umwälzen von Flüssigkeit vom unteren Teil des Reaktors (1) zum oberen Teil des Reaktors (1) oberhalb des Verpackungsmaterials;
vi) einen Auslass (6) zum Ablassen von Gas, in dem Kohlenmonoxid und/oder Kohlendioxid und Wasserstoff zu Methan umgewandelt wurden;
vii)einen Auslass (7) zum Ablassen von Flüssigkeit aus dem Reaktor (1), und
viii)einen Einlass (9) zum Einblasen von Druckluft in den Reaktor (1), wobei sich der Einlass (9) unterhalb des Verpackungsmaterials (2) befindet.

11. Vorrichtung nach Anspruch 10, wobei der Reaktor (1) eine Sprüheinrichtung (8) zum Besprühen der Flüssigkeit oberhalb des Verpackungsmaterials umfasst.

12. Vorrichtung nach Anspruch 11, wobei die Sprüheinrichtung (8) eine Steuereinrichtung zum Steuern der Durchflussrate der in den Reaktor gesprühten Flüssigkeit umfasst, so dass sie im Bereich von 0,5 bis 2,5 m³/m² der Querschnittsfläche des Reaktors liegt.

## Revendications

1. Procédé de conversion de monoxyde de carbone et/ou dioxyde de carbone et d'hydrogène dans un gaz en méthane, comprenant les étapes suivantes consistant à :
a) fournir un gaz comprenant du monoxyde de carbone et/ou du dioxyde de carbone ;
b) fournir un gaz comprenant de l'hydrogène ;
c) amener le gaz comprenant du monoxyde de carbone et/ou du dioxyde de carbone et le gaz comprenant de l'hydrogène à un réacteur, le réacteur comprenant un matériau de garnissage choisi dans le groupe constitué par le polyéthylène (PE), le polypropylène (PP), le polystyrène (PS) et l'acrylonitrile butadiène styrène (ABS) et le réacteur comprenant des micro-organismes capables de convertir le monoxyde de carbone et/ou le dioxyde de carbone et l'hydrogène en méthane, et le matériau de garnissage pouvant être détaché du réacteur ;
d) fournir un liquide et amener le liquide au réacteur au-dessus du matériau de garnissage ;
e) faire passer le gaz comprenant du monoxyde de carbone et/ou du dioxyde de carbone et le gaz comprenant de l'hydrogène à travers le matériau de garnissage comprenant le liquide et des micro-organismes pour convertir le monoxyde de carbone et/ou le dioxyde de carbone et l'hydrogène en méthane et eau ;
f) acheminer le gaz dans lequel le monoxyde de carbone et/ou le dioxyde de carbone et l'hydrogène sont convertis en méthane vers une sortie, et
g) nettoyer et décompresser le matériau de garnissage en remplissant le réacteur avec du liquide et en insufflant de l'air comprimé dans le réacteur au-dessous du matériau de garnissage.

2. Procédé selon la revendication 1, dans lequel les micro-organismes sont des micro-organismes méthanogènes.

3. Procédé selon l'une quelconque des revendications 1 et 2, dans lequel le liquide est choisi dans le groupe constitué par l'eau et un effluent d'un digesteur anaérobie.

4. Procédé selon l'une quelconque des revendications 1 à 3, dans lequel amener un liquide au réacteur se fait par aspersion continue.

5. Procédé selon la revendication 4, dans lequel l'aspersion continue s'effectue avec un débit du liquide dans la plage de 0,5 à 2,5 m³/m² de la surface de la section transversale du réacteur.

6. Procédé selon l'une quelconque des revendications 1 à 5, dans lequel le gaz comprenant du monoxyde de carbone et/ou du dioxyde de carbone est choisi dans le groupe constitué par le biogaz, le gaz de combustion et le gaz de synthèse.

7. Procédé selon l'une quelconque des revendications 1 à 6, dans lequel le gaz comprenant du monoxyde de carbone et/ou du dioxyde de carbone est un gaz ne contenant pas d'oxygène.

8. Procédé selon les revendications 1 à 7, dans lequel la température dans le réacteur est dans la plage de 40 °C à 70 °C.

9. Procédé selon la revendication 8, le procédé étant anaérobie.

10. Appareil destiné à convertir du monoxyde de carbone et/ou du dioxyde de carbone et de l'hydrogène dans un gaz en méthane, ledit appareil comprenant :
i) un réacteur (1) comprenant un matériau de garnissage (2), dans lequel le matériau de garnissage est choisi dans le groupe constitué par le polyéthylène (PE), le polypropylène (PP), le polystyrène (PS) et l'acrylonitrile butadiène styrène (ABS), et dans lequel des micro-organismes capables de convertir le monoxyde de carbone et/ou le dioxyde de carbone et l'hydrogène en méthane se développent à la surface du matériau de garnissage, et dans lequel le matériau de garnissage (2) peut être détaché du réacteur (1) ;
ii) une entrée (3) destinée à amener au réacteur (1) un gaz comprenant du monoxyde de carbone et/ou du dioxyde de carbone ;
iii) une entrée (4) destinée à amener au réacteur (1) un gaz comprenant de l'hydrogène ;
iv) une entrée (5) destinée à amener un liquide au réacteur (1).
v) un moyen (5A) destiné à faire circuler le liquide de la partie inférieure du réacteur (1) à la partie supérieure du réacteur (1) au-dessus du matériau de garnissage ;
vi) une sortie (6) destinée à l'évacuation de gaz dans lequel le monoxyde de carbone et/ou le dioxyde de carbone et l'hydrogène ont été convertis en méthane ;
vii) une sortie (7) destinée à l'évacuation de liquide à partir du réacteur (1), et
viii) une entrée (9) destinée à l'insufflation d'air comprimé dans le réacteur (1), l'entrée (9) étant située au-dessous du matériau de garnissage (2).

11. Appareil selon la revendication 10, dans lequel le réacteur (1) comprend un moyen d'aspersion (8) destiné à projeter le liquide au-dessus du matériau de garnissage.

12. Appareil selon la revendication 11, dans lequel le moyen d'aspersion (8) comprend un moyen de régulation destiné à réguler le débit du liquide projeté dans le réacteur pour qu'il se situe dans la plage de 0,5 à 2,5 m³/m² de la surface de la section transversale du réacteur.
